# EUROPEAN PATENT APPLICATION

(11) **EP 2 915 521 A1**
(43) Date of publication of application: **09.09.2015**
(21) Application number: 13851396.5
(22) Date of filing: 30.10.2013
(51) Int. Cl.: A61J 3/00, B65B 1/30, G06Q 50/22

(54) **DEVICE AND SYSTEM FOR PREPARING INDIVIDUALLY CUSTOMIZED NUTRITIONAL SUPPLEMENT**

(30) Priority: 30.10.2012 KR 20120121612
(71) Applicant: Aribio Inc., Seoul 150-095 (KR); An, Myeong Gu, Gyeonggi-do 446-719 (KR)
(72) Inventor: AN, Myeong Gu, Gyeonggi-do 446-719 (KR); SUNG, Soo-Hyun, Seoul 134-877 (KR)
(74) Representative: Reboussin, Yohann Mickaël Noël
(86) International application number: PCT/KR2013/009737
(87) International publication number: WO 2014/069896

(57) **Abstract**

Disclosed is an individualized nutrient preparation apparatus to prepare an individualized nutrient according to user characteristics. The individualized nutrient preparation apparatus includes a data processing unit configured to generate individual nutrient prescription data, a plurality of supply units configured to independently supply different nutrient granules according to the individual nutrient prescription data, each of which supplying a predetermined number set of the nutrient granules, and a packing unit configured to pack a mix of the nutrient granules.

## Description

### [Technical Field]

The present invention relates to a nutrient preparation apparatus, and more particularly to an individualized nutrient preparation apparatus which may prepare an individualized nutrient according to user characteristics.

### [Background Art]

In general, vitamins together with three major nutrient, such as protein, fat and carbohydrates, are substances necessary to adjust a physiological function even with a small amount and it is known that vitamins are incapable of being synthesized in a human body and thus need to be absorbed from the outside.

Recently, importance of vitamins is being more emphasized and the paradigm of vitamins is being changed. That is, vitamins were conventionally taken by persons suffering from vitamin deficiency so as to cure diseases caused thereby but are now used to prevent and cure various diseases as well as to supplement deficiency.

Further, indiscriminant overdose of vitamins may cause side effects and, thus, a person may take a proper dose of vitamins according to internal use purposes and individual characteristics. However, most consumers indiscriminately take many kinds of vitamins without consideration of health and correct understanding of medicines.

Therefore, several measures to prepare specialized nutrients individualized according to individual characteristics are proposed now. However, these measures are not yet satisfactory and development of new measures has been required.

### [Disclosure]

### [Technical Problem]

Therefore, the present invention has been made in view of the above problems, and it is an object of the present invention to provide an individualized nutrient preparation apparatus which may prepare an individualized nutrient according to user characteristics.

It is another object of the present invention to provide an individualized nutrient preparation apparatus which may prepare one dose of an individualized nutrient at a precise composition ratio according to individual nutrient prescription data.

It is yet another object of the present invention to provide an individualized nutrient preparation apparatus which may simplify a nutrient preparation process and rapidly prepare an individualized nutrient according to different prescription conditions.

### [Technical Solution]

In accordance with an aspect of the present invention, the above and other objects can be accomplished by the provision of an individualized nutrient preparation apparatus to prepare a nutrient according to user characteristics, including a data processing unit configured to generate individual nutrient prescription data, a plurality of supply units configured to independently supply different nutrient granules according to the individual nutrient prescription data, each of which supplying a predetermined number set of the nutrient granules, and a packing unit configured to pack a mix of the nutrient granules.

The data processing unit may generate the individual nutrient prescription data in various methods according requirements. For example, the data processing unit may be configured to generate the individual nutrient prescription data corresponding to medical inquiry data according to individual characteristics, input through an input unit.

Various terminals may be used as the input unit according to requirements. Here, terminals may include general electrical/electronic apparatuses, such as a desktop computer, a personal digital assistance (PDA), a smart phone, a handheld PC, a portable terminal, a notebook computer, a tablet computer and the like, but the present invention may not be limited as to kinds and characteristics of terminals.

For reference, in the present invention, the medical inquiry data may include data regarding user characteristics, for example, a health state, habits and the like, but the present invention may not be limited as to kinds of data included in medical inquiry data. For example, the medical inquiry data may include data regarding at least one of user's age, sex, weight, height, eating habits, drinking, smoking, labor type, possessing diseases and sleeping habits.

The data processing unit may generate the individual nutrient prescription data corresponding to the medical inquiry data input through the input unit. For example, the data processing unit may compare the medical inquiry data with predetermined reference data and calculate information regarding kinds and contents of specific nutrient ingredients required by an individual according to a result of comparison.

In the present invention, the reference data may be understood as data regarding kinds and doses of nutrient ingredients which need to be included in a diet required by healthy individuals and be predetermined in the data processing unit. For example, the reference data may be predetermined based on a single recommended dose. Further, contents of specific nutrient ingredients may be increased or decreased bas ed on medical inquiry data according to individual characteristics.

The generated individual nutrient prescription data may include information regarding kinds and contents of various nutrient ingredients specialized according to individual characteristics. For example, the individual nutrient prescription data may include the composition ratio of at least one of vitamins, minerals and amino acids.

For reference, in the present invention, supply of the nutrient granules one by one from the respective supply units may be understood as supply of one granule from each supply unit at the same time interval.

The number and disposition of the supply units may be variously modified according to requirements and design specifications. Although, for example, the supply units are disposed in a rectilinear shape, as circumstances require, the supply units may be disposed in a circular shape or other shapes.

The supply units may be provided in various structures which may supply nutrient granules one by one according to requirements or design specifications. For example, the supply unit may include a storage part to store nutrient granules and a discharge part to discharge the nutrient granules one by one from the storage part.

The structure and characteristics of the storage part may be variously modified. For example, the storage part may include a storage container and an opening/closing member to open and close an opening formed at the upper portion of the storage container. The opening/closing member may be provided on the storage container so as to be rotatable about one end thereof and thus to be opened and closed. As circumstances require, the opening/closing member may be opened and closed using a general sliding method, but the present invention may not be limited as to the opening and closing method of the opening/closing member.

Further, guide plates to define a flow path of the nutrient granules may be provided within the storage container and the nutrient granules may be guided to the discharge part along the guide plates. For example, a plurality of guide plates may be alternately disposed at different heights and separated from one another in the vertical direction, thus being capable of defining a flow path of a zigzag shape.

As another example, the storage part may include a storage container and a partition member configured to divide the inner space of the storage container into a storage space to store nutrient granules and a reception space to receive a dehumidifying agent and provided with communication slots to communicate the storage space and the reception space with each other.

Further, a storage amount sensor configured to sense the storage amount of nutrient granules stored in the storage container and a warning generation part configured to output a warning signal if a value measured by the storage amount sensor is less than a predetermined value may be provided on the storage container.

The discharge part may be provided in various structures which may discharge the nutrient granules, supplied from the storage part, one by one.

As one example, the discharge part may include a pickup plate including receipt grooves formed along the outer circumferential surface and configured to be rotatable vertically so as to be adjacent to an outlet of the storage part, and a guide member including a granule discharge hole and configured to surround the outer circumferential surface of the pickup plate, and the nutrient granules may be picked up one by one by the receipt grooves as the pickup plate is vertically rotated and, if the granule discharge hole is disposed so as to coincide with the receipt groove in the vertical direction when the pickup plate is rotated, the nutrient granule received in the receipt groove may be discharged through the granule discharge hole.

As another example, the discharge part may include a first plate including receipt grooves to receive the nutrient granules supplied from the storage part one by one and a second plate including a granule discharge hole and provided under the first plate so as to be rotatable relative to the first plate, and, if the granule discharge hole is disposed so as to coincide with the receipt groove in the vertical direction during relative rotation between the first plate and the second plate, the nutrient granule received in the receipt groove may be discharged through the granule discharge hole.

For reference, relative rotation of the second plate to the first plate may be understood as including rotation of the second plate in the fixed state of the first plate and rotation of the first plate in the fixed state of the second plate.

Further, the discharge part may include a discharge member configured to discharge the nutrient granules so that the nutrient granules may be more effectively discharged. For example, the discharge member may be provided adjacent to the granule discharge hole and configured to discharge the nutrient granule from the receipt groove approaching the granule discharge hole. For this purpose, guide grooves communicating with the receipt grooves may be formed on the first plate, a part of the discharge member may be received in the guide grooves, and the nutrient granule received in the receipt groove approaching the granule discharge hole may be pressed by the discharge member and discharged.

The individualized nutrient preparation apparatus may further a first sensor provided adjacent to one end of the granule discharge hole and a second sensor provided adjacent to the other end of the granule discharge hole, the first sensor may sense whether or not the receipt groove approaching the granule discharge hole receives the nutrient granule, and the second sensor may sense whether or not the receipt groove passing through the granule discharge hole discharges the nutrient granule.

As another example, the discharge part may include a pickup roller including pickup holes and configured to be rotatable adjacent to the outlet of the storage part and an actuator configured to provide driving force to rotate the pickup roller, and, as the pickup roller is rotated, and the nutrient granules may be picked up one by one by the pickup holes and then discharged.

The pickup holes may be formed to have a size to pick up one nutrient granule at a time and the number and separation interval of pickup holes may be variously modified. For example, the pickup holes may have a size corresponding to the nutrient granules or a smaller size (diameter) than the nutrient granules.

Various types of actuators may be used as the actuator according to requirements and design specifications, but the present invention may not be limited as to the connection structure between actuator and the pickup roller. For example, the actuator may be configured to provide rectilinear driving force and an interlock member to convert the rectilinear driving force into rotary driving force may be connected to the pickup roller. As circumstances require, a separate connection member may be excluded and the pickup roller may be rotated directly by the driving force of the actuator.

The discharge part may include a transfer guide part configured to transfer the nutrient granules picked up by the pickup roller and a granule transfer hole to transfer the nutrient granules one by one may be formed on the transfer guide part. Further, a temporary loader part to temporarily load the nutrient granules prior to pickup may be formed on the transfer guide part and the nutrient granules supplied from the storage part and loaded on the temporary loader part may be picked up one by one by the pickup roller and transferred through the granule transfer hole.

Further, the discharge part may include a vacuum suction part and the nutrient granules may be sucked by the pickup hole by vacuum suction force. Further, a vacuum pressure sensor to sense the vacuum pressure of the vacuum suction part may be provided and whether or not the nutrient granule is normally picked up may be sensed using a signal sensed by the vacuum pressure sensor. For example, when the vacuum pressure sensor senses a predetermined pressure, it may be judged that the nutrient granule is normally picked up through the pickup hole and, when the vacuum pressure sensor senses a pressure lower than the predetermined pressure, it may be judged that the nutrient granule is abnormally picked up through the pickup hole. As circumstances require, the vacuum suction part may be excluded and the nutrient granules may be picked up one by on simply by the pickup roller.

The vacuum suction part may be individually provided on each of the supply units or the supply units may commonly use one vacuum suction part. For example, the vacuum suction part and the pickup rollers of the respective supply units may be independently connected by a plurality of connection channels, and control valves to selectively cut off independently at least one of the respective connection channels may be provided on the respective connection channels.

A hopper configured to gather the nutrient granules supplied from the respective supply units into one place may be provided under the supply units and the nutrient granules supplied from the supply units to the hopper may be gathered along the inclined guide surface of the hopper and guided to the packing unit.

The packing unit may be provided to pack the gathered nutrient granules into units of one dose according to requirements and design specifications. For example, the packing unit may pack the nutrient granules into at least one type of a capsule, a packet and a stick, but the present invention may not be limited as to the packing shape and type of the packing unit.

In accordance with another aspect of the present invention, there is provided an individualized nutrient preparation system to prepare a nutrient according user characteristics, including an input unit configured to input medical inquiry data according to individual characteristics, a data processing unit configured to generate individual nutrient prescription data corresponding to the medical inquiry data, and a preparation unit configured to receive the individual nutrient prescription data and prepare the nutrient according to the individual nutrient prescription data.

### [Advantageous Effects]

As described above, an individualized nutrient preparation apparatus in accordance with the present invention may prepare an individualized nutrient according user characteristics.

Particularly, in accordance with the present invention, different supply units may supplies different nutrient granules one by one as the predetermined number set to one dose according to individual nutrient prescription data and thus, one dose of the individual nutrient may be prepared at a precise composition ratio.

Further, in accordance with the present invention, if a granule discharge hole is disposed so as to coincide with a receipt groove in the vertical direction during rotation of a pickup plate (or relative rotation between a first plate and a second plate), a nutrient granule received in the receipt groove may be discharged through the granule discharge hole and thus, excessive supply of specific nutrient granules may be prevented and nutrient granules may be more stably and precisely supplied one by one.

Further, in accordance with the present invention, the nutrient granules are picked up one by one in a vacuum suction method and then supplied and thus, excessive supply of specific nutrient granules may be prevented and nutrient granules may be more stably and precisely supplied one by one.

Further, in accordance with the present invention, different supply units may supplies different nutrient granules one by one as the predetermined number according to individual nutrient prescription data and thus, a nutrient preparation process may be simplified and a nutrient may be rapidly prepared according to different prescription conditions.

Further, in accordance with the present invention, an individualized nutrient specialized according to individual characteristics, such as user's age, sex, weight, height, eating habits, drinking, smoking, labor type, possessing diseases and sleeping habits, may be prepared.

Moreover, the individualized nutrient preparation apparatus in accordance with the present invention may prevent indiscriminate taking nutrients and side effects thereby and contribute to promotion of user health.

### [Description of Drawings]

FIG. 1 is a front view illustrating an individualized nutrient preparation apparatus in accordance with the present invention.
FIG. 2 is a plan view illustrating the individualized nutrient preparation apparatus in accordance with the present invention.
FIGs. 3 and 4 are views illustrating a supply unit and a storage part of the individualized nutrient preparation apparatus in accordance with the present invention.
FIGs. 5 to 7 are views illustrating a modification of the supply unit and the storage part of the individualized nutrient preparation apparatus in accordance with the present invention.
FIG. 8 is a view illustrating a packing unit of the individualized nutrient preparation apparatus in accordance with the present invention.
FIG. 9 is a view illustrating nutrients prepared by the individualized nutrient preparation apparatus in accordance with the present invention.
FIG. 10 is a view illustrating an individualized nutrient preparation apparatus in accordance with another embodiment of the present invention.
FIG. 11 is a view illustrating a supply unit of the individualized nutrient preparation apparatus in accordance with another embodiment of the present invention.
FIG. 12 is a view illustrating a storage part of the individualized nutrient preparation apparatus in accordance with another embodiment of the present invention.
FIG. 13 is a view illustrating a modification of the storage part of the individualized nutrient preparation apparatus in accordance with another embodiment of the present invention.
FIGs. 14 and 15 are views illustrating an operating structure of the supply unit of the individualized nutrient preparation apparatus in accordance with another embodiment of the present invention.
FIG. 16 is a view illustrating a vacuum suction part of the individualized nutrient preparation apparatus in accordance with another embodiment of the present invention.
FIG. 17 is a view illustrating a modification of the supply unit of the individualized nutrient preparation apparatus in accordance with another embodiment of the present invention.
FIGs. 18 to 20 are views illustrating a process for calculating individual nutrient prescription data according to user characteristics using an individualized nutrient preparation apparatus in accordance with the present invention.

### [Best Mode]

Hereinafter, preferred embodiments of the present invention will be described in detail with reference to accompanying drawings. In the following description of the present invention, a detailed description of known functions and configurations incorporated herein will be omitted when it may make the subject matter of the present invention rather unclear.

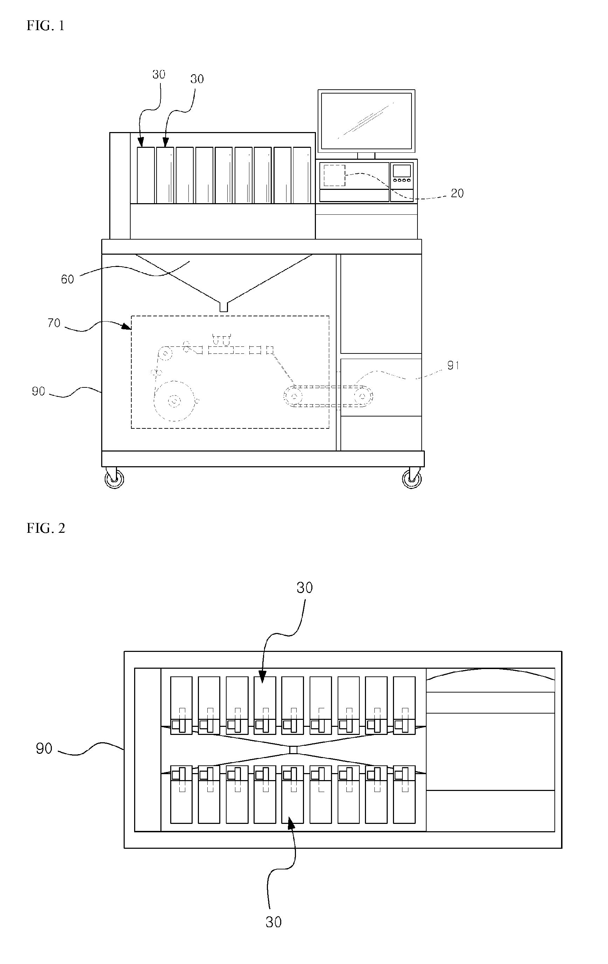

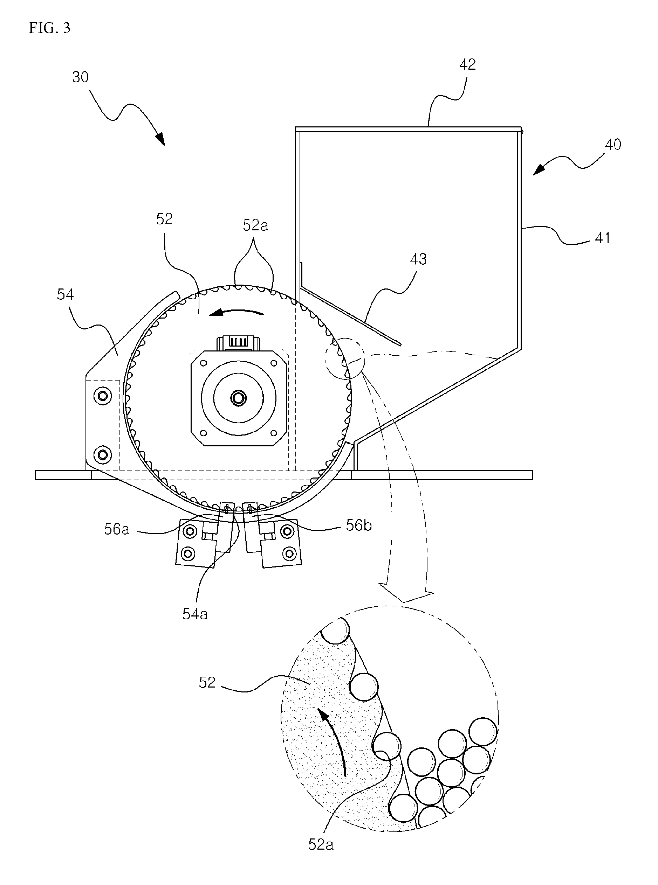

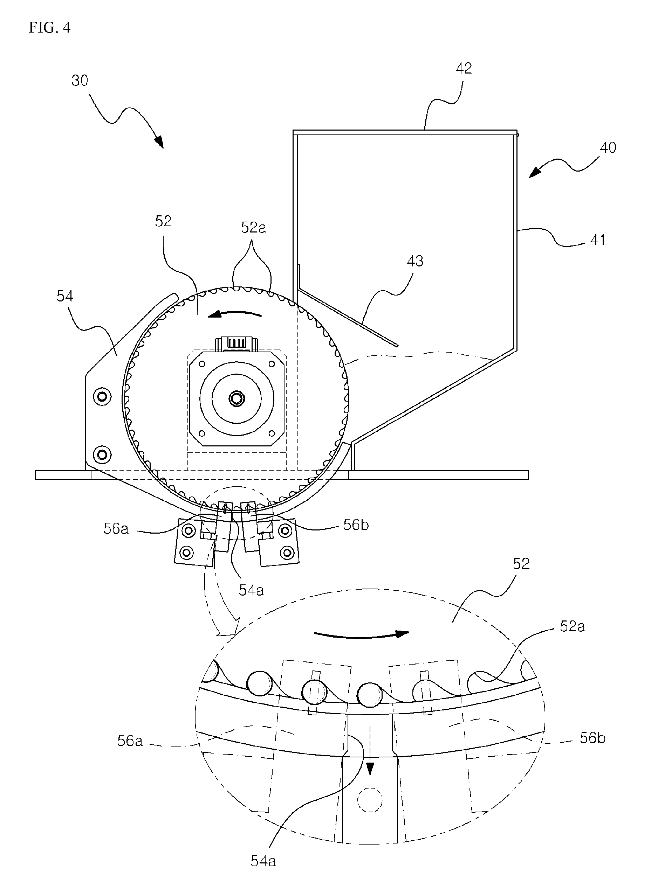

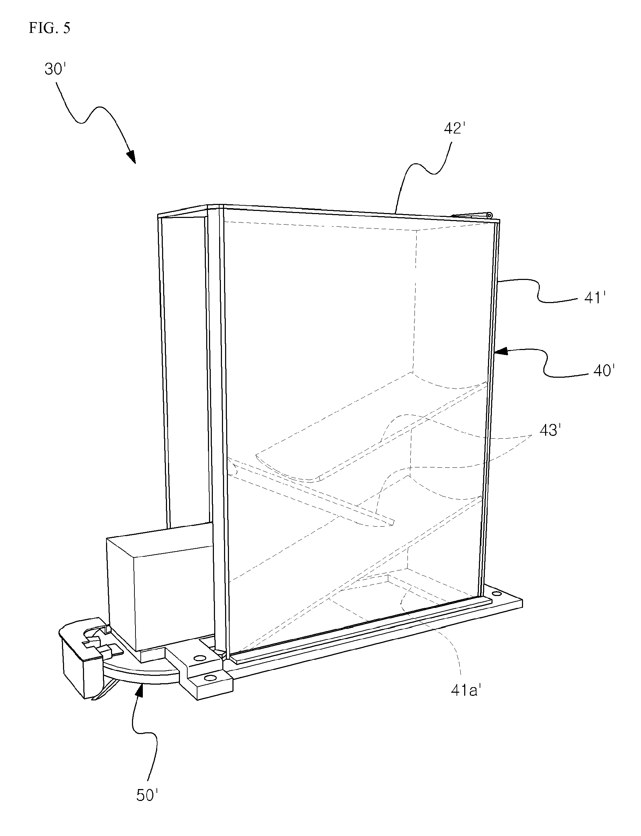

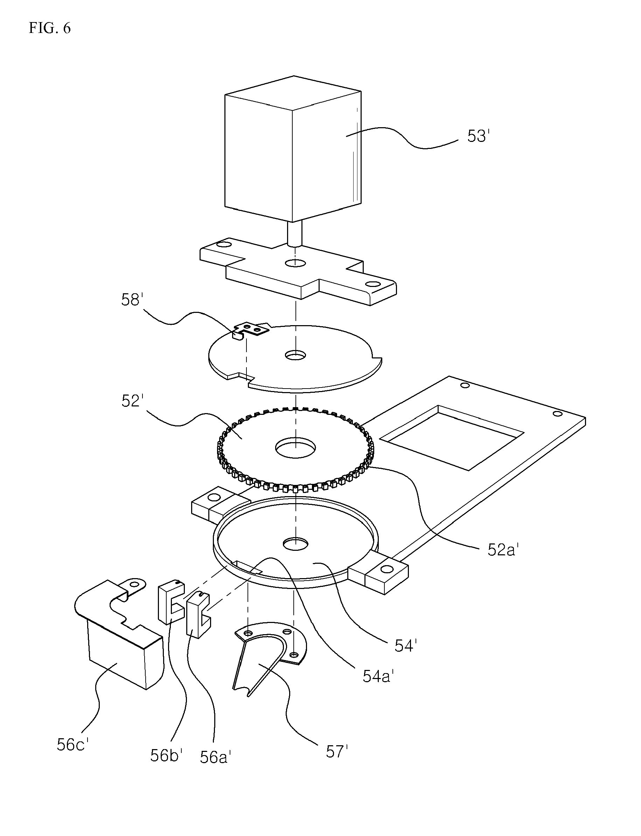

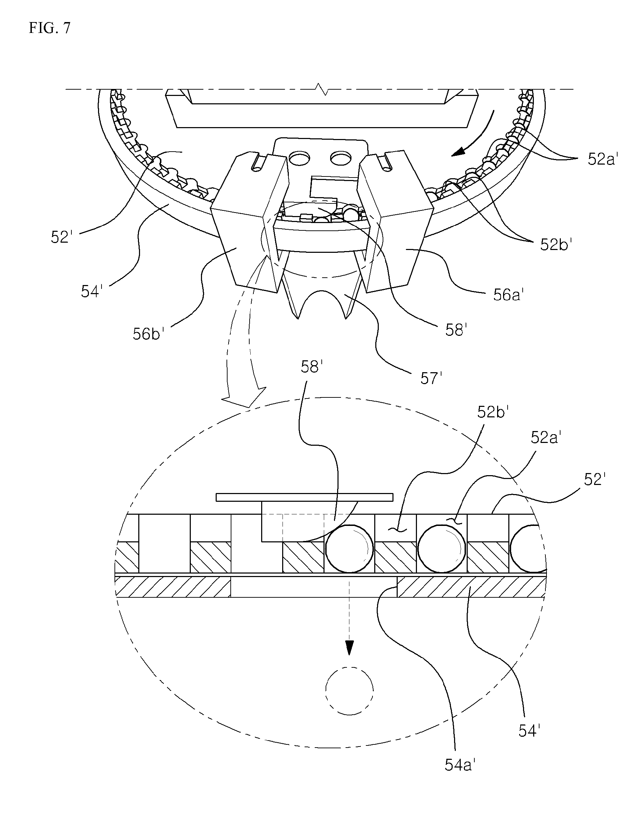

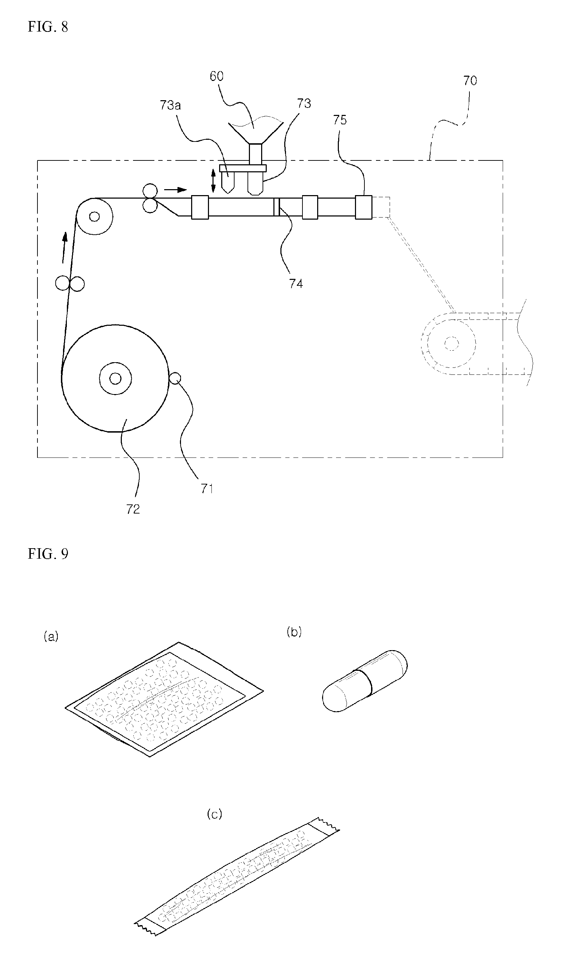

FIG. 1 is a front view illustrating an individualized nutrient preparation apparatus in accordance with the present invention, FIG. 2 is a plan view illustrating the individualized nutrient preparation apparatus in accordance with the present invention, FIGs. 3 and 4 are views illustrating a supply unit and a storage part of the individualized nutrient preparation apparatus in accordance with the present invention, FIGs. 5 to 9 are views illustrating a modification of the supply unit and the storage part of the individualized nutrient preparation apparatus in accordance with the present invention, FIG. 8 is a view illustrating a packing unit of the individualized nutrient preparation apparatus in accordance with the present invention, and FIG. 9 is a view illustrating nutrients prepared by the individualized nutrient preparation apparatus in accordance with the present invention.

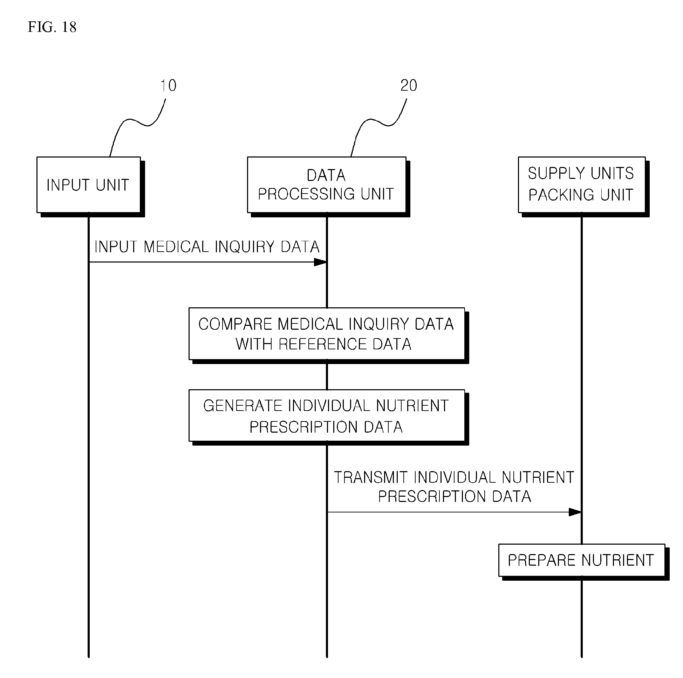

FIGs. 18 to 20 are views illustrating a process for calculating individual nutrient prescription data according to user characteristics using an individualized nutrient preparation apparatus in accordance with the present invention.

As exemplarily shown in these figures, an individualized nutrient preparation apparatus in accordance with the present invention includes a data processing unit 20 and a preparation unit and is configured so as to prepare an individualized nutrient specialized according to user characteristics.

The data processing unit 20 is provided so as to generate individual nutrient prescription data. For example, with reference to FIG. 18, the data processing unit 20 may be configured so as to generate individual nutrient prescription data corresponding to medical inquiry data input through an input unit 10.

That is, the input unit 10 may be provided so as to input medical inquiry data according to individual characteristics. Various terminals may be used as the input unit 10 according to requirements. Here, terminals may include general electrical/electronic apparatuses, such as a desktop computer, a personal digital assistance (PDA), a smart phone, a handheld PC, a portable terminal, a notebook computer, a tablet computer and the like, but the present invention is not limited as to kinds and characteristics of terminals.

For reference, in the present invention, medical inquiry data may include data regarding user characteristics, for example, a health state, habits and the like, but the present invention is not limited as to kinds of data included in medical inquiry data.

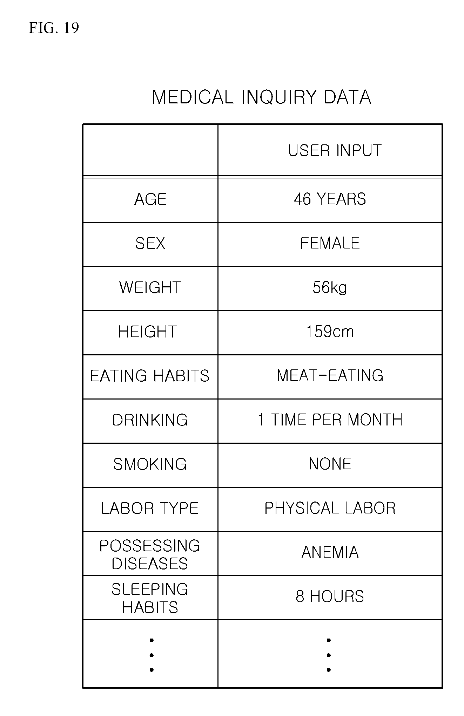

For example, with reference to FIG. 19, medical inquiry data may include data regarding at least one of user's age, sex, weight, height, eating habits, drinking, smoking, labor type, possessing diseases and sleeping habits.

Although this embodiment of the present invention exemplarily illustrates respective information of medical inquiry data as being input as a shortly answering type, the respective information may be segmented according to requirements. For example, information regarding eating habits may include an item to select eating-meat or eating-vegetables and segmented information regarding daily intake, information regarding smoking may include segmented information regarding an amount of smoking per day, and information regarding drinking may include segmented information regarding kinds of alcohols and an amount of drinking once.

The data processing unit 20 is provided to generate individual nutrient prescription data corresponding to medical inquiry data input through the input unit 10. That is, when medical inquiry data according to individual characteristics is input, the data processing unit 20 may generate individual nutrient prescription data specialized according to individual characteristics based on the input medical inquiry data.

For example, the data processing unit 20 may compare the medical inquiry data with predetermined reference data and calculate information regarding kinds and contents of specific nutrient ingredients required by an individual according to a result of comparison.

In the present invention, the reference data may be understood as data regarding kinds and doses of nutrient ingredients which need to be included in a diet required by healthy individuals and be predetermined in the data processing unit 20. For example, the reference data may be predetermined based on a single recommended dose. Further, contents of specific nutrient ingredients may be increased or decreased based on medical inquiry data according to individual characteristics. For example, although a single recommended dose of a specific nutrient ingredient corresponding to a specific age and sex is 5mg, the dose of the specific nutrient ingredient may be increased or decreased according to different individual characteristics (for example, eating habits, possessing diseases and the like) so as to be greater or less than 5 mg. Differently, the reference data may be predetermined based on a recommended daily dose.

The input unit 10 and the data processing unit 20 may be connected so that they may execute remote communication. Hereinafter, connection of the input unit and the data processing unit 20 via the Internet will be exemplarily described. As circumstances require, the input unit and the data processing unit may be connected through other different communication networks, such as a local area network (LAN), a packet data network (PDN), and a public switched telephone network, so that they may execute remote communication.

The individual nutrient prescription data generated by the above-described procedure may include information regarding kinds and contents of various nutrient ingredients specialized according to individual characteristics. For example, the individual nutrient prescription data may include the composition ratio of at least one of vitamins, minerals and amino acids.

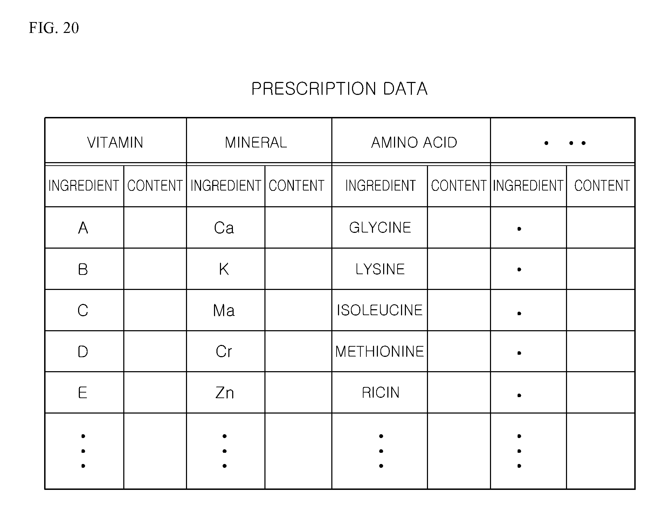

With reference to FIG. 20, the individual nutrient prescription data may include information regarding various vitamin ingredients, including vitamin A, vitamin B, vitamin C, vitamin D and vitamin E, and contents of these vitamin ingredients. Further, information regarding vitamin B may include segmented information regarding vitamin B1 (thiamine), vitamin B2 (riboflavin), vitamin B3 (niacin, nicotine amid) and vitamin B4 (pantothenic acid).

Further, the individual nutrient prescription data may include information regarding various mineral ingredients, such as calcium (Ca), potassium (K), magnesium (Mg), chrome (Cr), zinc (Zn) and the like, and contents of these mineral ingredients and include information regarding various amino acids, such as glycine, lysine, isoleucine, methionine, ricin and the like, and contents of these amino acids.

The individual nutrient prescription data generated by the data processing unit 20 may be transmitted directly to the preparation unit, which will be described later, and, as circumstances require, the individual nutrient prescription data generated by the data processing unit may be transmitted to the input unit (for example, a user terminal) and then transmitted to the preparation unit.

Further, the individual nutrient prescription data may be transmitted as a document or file which may be confirmed by general users and, as circumstances require, be transmitted as an encrypted document or file which may be confirmed by a specific user. Otherwise, the individual nutrient prescription data may be converted into a QR code and then transmitted.

As described above, in the present invention, individual nutrient prescription data specialized according to individual characteristics, such as user's age, sex, weight, height, eating habits, drinking, smoking, labor type, possessing diseases and sleeping habits, may be generated. For example, a prescription of a nutrient including calcium, magnesium, vitamin D and the like to prevent osteoporosis may be given to a woman past menopause and contents of respective ingredients of the nutrient may be properly increased or decreased according to different individual characteristics, such as user's age, sex, weight, height, eating habits, drinking, smoking, labor type, possessing diseases and sleeping habits.

With reference to FIGs. 1 to 9, the preparation unit is configured to receive individual nutrient prescription data and to prepare a nutrient according to the individual nutrient prescription data, and includes supply units 30 and a packing unit 70. Hereinafter, the data processing unit 20, the supply units 30 and the packing unit 70, which are mounted on an dedicated table 90, will be exemplarily described.

The table 90 may have various shapes and structures according to requirements and design specifications, but the present invention is not limited as to the shape and structure of the table 90. For example, the table 90 may have an approximately rectangular box shape, a plurality of supply units 30, which will be described later, may be disposed on the upper surface of the table 90, and the packing unit 70, which will be described later, may be disposed within the table 90. Further, the front surface of the table 90 may be formed of a transparent material so that a worker may easily observe the state of the inside of the table 90.

A discharge conveyer 91 to discharge a nutrient, packed into designated units by the packing unit 70, may be formed at one side of the inside of the table 90, and an input/output unit (not shown) to output various pieces of information, such as a general progressing state (for example, a supply amount, a remaining amount, a supply ratio and the like) and a preparation environment (for example, humidity, temperature and the like), and to receive inputs for execution of specific functions may be provided at the other side of the upper portion of the table 90. For example, the input/output unit may be provided as a general touchscreen, but the present invention is not limited as to the kind and characteristics of the input/output unit.

The supply units 30 are provided to independently supply different kinds of nutrient granules one by one according to individual nutrient prescription data. Here, supply of nutrient granules one by one from the respective supply units 30 may be understood as supply of one granule from each supply unit 30 at the same time interval.

Further, in the present invention, nutrient granules may include at least one of vitamins, minerals, amino acids, a Korean traditional medicine, and other nutrients, but the present invention is not limited as to kinds and characteristics of nutrient granules. For reference, nutrient granules may have a size (or a diameter) of about 0.5 mm~2mm and, as circumstances require, may have a size smaller or greater than 0.5 mm~2mm.

The number and disposition of the supply units 30 may be variously modified according to requirements and design specifications. Hereinafter, the supply units 30 disposed in two straight lines in parallel on the upper surface of a hopper 60, which will be described later, will be exemplarily described. As circumstances require, the supply units 30 may be disposed in a circular shape or other shapes, but the present invention is not limited as to the disposition of the supply units.

The supply units 30 are configured to supply different kinds of nutrient granules one by one according to individual nutrient prescription data. Hereinafter, the supply units 30 configured such that each of the supply units 30 supplies different nutrient granules one by one as the predetermined number set to one dose will be exemplarily described. For example, one dose of a nutrient prescribed according to specific prescription data includes 5 granules of an ingredient A, 3 granules of an ingredient B and 2 granules of an ingredient C, the supply unit A 30 may supply a granule of the ingredient A 5 times, the supply unit B 30 may supply a granule of the ingredient B 3 times, and the supply unit C 30 may supply a granule of the ingredient C 2 times. As circumstances require, the respective supply units may be configured to supply nutrient granules one by one up to two or three doses.

The supply units 30 may have various structures which may supply nutrient granules one by one according to requirements or design specifications. Hereinafter, the supply unit 30 including a storage part 40 to store nutrient granules and a discharge part 50 to discharge the nutrient granules from the storage part 40 one by one will be exemplarily described.

As one example, with reference to FIGs. 3 and 4, the storage part 40 may be fixedly installed on the dedicated table 90. Hereinafter, the storage part 40 including a storage container 41 and an opening/closing member 42 will be exemplarily described.

The storage container 41 may be formed in various shapes, such as a circular shape, a rectangular shape or a polygonal shape, according to requirements and design specifications. An opening may be formed at the upper portion of the storage container 41 and a discharge hole (not shown) to discharge nutrient granules may be formed at the lower portion of the storage container 41.

The opening/closing member 42 may be provided to open and close the opening formed at the upper portion of the storage container 41. For example, the opening/closing member 42 may be provided at the upper portion of the storage container 41 so as to be rotatable about one end thereof and thus to be opened and closed. As circumstances require, the opening/closing member may be opened and closed using a general sliding method, but the present invention is not limited as to the opening and closing method of the opening/closing member.

If nutrient granules stored in the storage container 41 are less than a designated amount, the opening/closing member 42 may be opened so as to fill the storage container 41 with nutrient granules.

Further, a guide plate 43 to define a flow path of the nutrient granules may be provided within the storage container 41 and the nutrient granules may be guided to the discharge part 50 along the flow path defined by the guide plate 43.

Further, the storage container 41 may be provided with an air blowing device (not shown) to dehumidify the nutrient granules and a means of filtering (not shown) to prevent influx of moisture in the air.

The discharge part 50 may be formed in various structures which may discharge nutrient granules, supplied from the storage part 40, one by one. For example, the discharge part 50 may include a pickup plate 52 and a guide member 54.

The pickup plate 52 may be formed in a disk shape having a designated diameter and be rotated vertically adjacent to an outlet of the storage part 40. Here, vertical rotation of the pickup plate 52 may be understood as rotation of the pickup plate 52 about a rotary shaft disposed in parallel with the ground surface.

A plurality of receipt grooves 52a to receive the respective nutrient granules supplied from the storage part 40 is formed along the outer circumferential surface of the pickup plate 52. For example, a plurality of receipt grooves 52a separated in the circumferential direction may be formed on the edge of the circumference of the first plate 52. The number and separation interval of the receipt grooves may be properly modified according to requirements and design specifications.

The guide member 54 may be provided to surround the outer circumferential surface of the pickup plate 52 and a granule discharge hole 54a corresponding to the receipt groove 52a may be formed on the guide member 54. For example, the guide member 54 may be formed in an about "C" shape so as to surround a part of the outer circumferential surface of the pickup plate 52. Further, the granule discharge hole 54a may be formed in a greater size than the receipt grooves 52a so that a nutrient granule may smoothly pass through the granule discharge hole 54a. As circumstances require, the receipt grooves and the granule discharge hole may be formed in the same size and shape.

For reference, the pickup plate 52 may be rotated by a general driving motor. The driving motor 53 may employ various general motors, such as a stamping motor, a servo motor, a stepper motor, a DC motor, an AC motor and the like, but the present invention is not limited as to the kind and characteristics of the driving motor.

By the above structure, as the pickup plate 52 is rotated vertically, the receipt grooves 52a formed on the outer circumferential surface of the pickup plate 52 may pick up nutrient granules one by one. When the pickup plate 52 is rotated in such a state in which the receipt grooves 52a pick up the nutrient granules, if the granule discharge hole 54a is disposed so as to coincide with the receipt groove 52a in the vertical direction, the nutrient granule received in the receipt groove 52a may be discharged through the granule discharge hole 54a. That is, in a state in which the granule discharge hole 54a is disposed so as not to coincide with the receipt groove 52a, the lower portion of the receipt groove 52a is blocked by the guide member 54 and thus the nutrient granule received in the receipt groove 52a may not be discharged, and, only in a state in which the granule discharge hole 54a is disposed so as to coincide with the receipt groove 52a in the vertical direction, the nutrient granule received in the receipt groove 52a may be dropped through the granule discharge hole 54a and discharged to the outside.

The amount of nutrient granules discharged by the discharge part 50 may be properly modified by adjusting the rotational velocity of the pickup plate 52 or changing the number of the receipt grooves 52a. Further, the amount of nutrient granules discharged by the discharge part 50 may be calculated using the rotational velocity of the pickup plate 52 or the number of the receipt grooves 52a or detected through separate sensors.

Hereinafter, sensing of the amount of the discharged nutrient granules using a first sensor 56a and a second sensor 56b will be exemplarily described.

The first sensor 56a may be provided adjacent to one end of the granule discharge hole 54a and the second sensor 56b may be provided adjacent to the other end of the granule discharge hole 54a. The first sensor 56a may sense whether or not the receipt groove 52a approaching the granule discharge hole 54a receives a nutrient granule and the second sensor 56b may sense whether or not the receipt groove 52a passing through the granule discharge hole 54a discharges a nutrient granule. That is, the first sensor 56a may sense whether or not the receipt groove 52a approaching the granule discharge hole 54a normally receives a nutrient granule and the second sensor 56b may sense whether or not the receipt groove 52a passing through the granule discharge hole 54a normally discharges a nutrient granule, thus being capable of precisely counting the amount of discharged nutrient granules.

The first sensor 56a and the second sensor 56b may employ a general photo sensor, beam sensor, contact sensor and the like, but the present invention is not limited as to the kinds and characteristics of the sensors.

The discharge part using the pickup plate which is rotated vertically may minimize generation of dust and damage due to friction between nutrient granules and minimize jamming of nutrient granules between the pickup plate and the guide member during rotation of the pickup plate.

As another example, with reference to FIGs 5 to 7, a storage part 40' may be fixedly installed on an dedicated table 90'. Hereinafter, the storage part 40' including a storage container 41' and an opening/closing member 42' will be exemplarily described.

The storage container 41' may be formed in various shapes, such as a circular shape, a rectangular shape or a polygonal shape, according to requirements and design specifications. An opening may be formed at the upper portion of the storage container 41' and a discharge hole (not shown) to discharge nutrient granules may be formed at the lower portion of the storage container 41'.

The opening/closing member 42' may be provided to open and close the opening formed at the upper portion of the storage container 41'. For example, the opening/closing member 42' may be provided at the upper portion of the storage container 41' so as to be rotatable about one end thereof to be opened and closed. As circumstances require, the opening/closing member may be opened and closed using a general sliding method, but the present invention is not limited as to the opening and closing method of the opening/closing member.

If nutrient granules stored in the storage container 41' are less than a designated amount, the opening/closing member 42' may be opened so as to fill the storage container 41 with nutrient granules.

Further, guide plates 43' to define a flow path of the nutrient granules may be provided within the storage container 41' and the nutrient granules may be guided to the discharge part 50' along the flow path defined by the guide plates 43'.

The structure and disposition of the guide plates 43' may be variously modified according to requirements and design specifications. For example, a plurality of guide plates 43' may be alternately disposed at different heights and separated from one another in the vertical direction, thus being capable of defining a flow path of a zigzag shape. By such a structure, nutrient granules put into the storage containers 41 ' may flow along the respective guide plates 43' and be transmitted to the discharge part 50'. Further, since the nutrient granules put into the storage container 41' may flow in the zigzag shape along the respective guide plates 43', clogging of the flow path with the nutrient granules due to a drop speed and friction may be prevented.

Further, the storage container 41' may be provided with a ventilation hole 41a' to prevent a supply defect of nutrient granules due to humidity change. For example, the ventilation hole 41a' may be formed at one side of the lower surface of the storage container 41'. As circumstances require, the ventilation hole may be formed on the side surface or the upper surface of the storage container and the ventilation hole may be configured to be selectively opened and closed.

The discharge part 50' may be formed in various structures which may discharge nutrient granules, supplied from the storage part 40', one by one. For example, the discharge part 50' may include a first plate 52' and a second plate 54'.

The first plate 52' may be formed in a disk shape having a designated diameter and receipt grooves 52a to receive the respective nutrient granules supplied from the storage part 40 are formed on the first plate 52'. For example, a plurality of receipt grooves 52a' separated in the circumferential direction may be formed on the edge of the circumference of the first plate 52'. The number and separation interval of the receipt grooves may be properly modified according to requirements and design specifications.

The second plate 54' may be provided under the first plate 52' so as to be rotatable relative to the first plate 52' and a granule discharge hole 54a' corresponding to the receipt groove 52a' is formed on the second plate 54'. For example, the granule discharge hole 54a may be formed in a curved slot shape having a greater length than the receipt grooves 52a' so that a nutrient granule may smoothly pass through the granule discharge hole 54a'. As circumstances require, the receipt grooves and the granule discharge hole may be formed in the same size and shape.

For reference, relative rotation of the second plate 54' to the first plate 52' may be understood as including rotation of the second plate in the fixed state of the first plate and rotation of the first plate in the fixed state of the second plate. Hereinafter, rotation of the first plate 52' by a general driving motor 53' in the fixed state of the second plate 54' will be exemplarily described. Various general motors, such as a stamping motor, a servo motor, a stepper motor, a DC motor, an AC motor and the like, may be used as the driving motor 53', but the present invention is not limited as to the kind and characteristics of the driving motor.

By the above structure, during relative rotation between the first plate 52' and the second plate 54', if the granule discharge hole 54a' is disposed so as to coincide with the receipt groove 52a' in the vertical direction, a nutrient granule received in the receipt groove 52a' may be discharged through the granule discharge hole 54a'. That is, in a state in which the granule discharge hole 54a' is disposed so as not to coincide with the receipt groove 52a', the lower portion of the receipt groove 52a' is blocked by the second plate 54' and thus the nutrient granule received in the receipt groove 52a' may not be discharged, and, only in a state in which the granule discharge hole 54a' is disposed so as to coincide with the receipt groove 52a' in the vertical direction, the nutrient granule received in the receipt groove 52a' may be dropped through the granule discharge hole 54a' and discharged to the outside.

Further, the discharge part 50' may include a discharge member 58' to discharge nutrient granules so that the nutrient granules may be more effectively discharged.

Hereinafter, provision of the discharge member 58' adjacent to the granule discharge hole 54a' and discharge of the nutrient granule from the receipt groove 52a' approaching the granule discharge hole 54a' by the discharge member 58' will be exemplarily described. For reference, the receipt groove 52a' approaching the granule discharge hole 54a' may be understood as a receipt groove most closely approaching the granule discharge hole 54a' from among the receipt grooves 52a', as the first plate 52' is rotated.

For example, guide grooves 52b' communicating with the receipt grooves 52a' may be formed on the first plate 52' and a part of the lower end of the discharge member 58' may be received in the guide grooves 52b'. Therefore, a nutrient granule received in the receipt groove 52a' approaching the granule discharge hole 54a' may be pressed by the discharge member 58' and discharged.

Such a structure may firmly discharge a nutrient granule under the condition that it is difficult to normally discharge the nutrient granule. For example, even if a nutrient granule is stuck to the inside of the receipt groove, the nutrient granule may be pressed by the discharge member and thus firmly discharged. As circumstances require, a spring member, a solenoid, a rotating member and the like may be used as the discharge member, but the present invention is not limited as to the kind and characteristics of the discharge member.

The amount of nutrient granules discharged by the discharge part 50' may be properly modified by adjusting the rotational velocity of the first plate 52' or changing the number of the receipt grooves 52a'. Further, the amount of nutrient granules discharged by the discharge part 50' may be calculated using the rotational velocity of the first plate 52' or the number of the receipt grooves 52a' or detected through separate sensors.

Hereinafter, sensing of the amount of discharged nutrient granules using a first sensor 56a' and a second sensor 56b' will be exemplarily described.

The first sensor 56a' may be provided adjacent to one end of the granule discharge hole 54a' and the second sensor 56b' may be provided adjacent to the other end of the granule discharge hole 54a'. The first sensor 56a' may sense whether or not the receipt groove 52a' approaching the granule discharge hole 54a' receives a nutrient granule and the second sensor 56b' may sense whether or not the receipt groove 52a passing through the granule discharge hole 54a' discharges a nutrient granule. That is, the first sensor 56a' may sense whether or not the receipt groove 52a' approaching the granule discharge hole 54a' normally receives a nutrient granule and the second sensor 56b' may sense whether or not the receipt groove 52a' passing through the granule discharge hole 54a' normally discharges a nutrient granule, thus being capable of precisely counting the amount of discharged nutrient granules.

The first sensor 56a' and the second sensor 56b' may employ a general photo sensor, beam sensor, contact sensor and the like, but the present invention is not limited as to the kinds and characteristics of the sensors. Further, a cover member 56c' to protect the first sensor 56a' and the second sensor 56b' may be provided on the second plate.

Further, the hopper 60 to gather nutrient granules supplied from the respective supply units 30 into one place may be provided under the supply units 30. The respective nutrient granules supplied from the supply units 30 to the hopper 60 may be guided to the center of the hopper 60 along the inclined guide surface of the hopper 60 and the gathered nutrient granules may be guided to the packing unit 70.

Further, a cleaning unit (not shown) to clean the hopper 60 may be provided on the hopper 60. For example, the cleaning unit may be configured so as to clean the hopper 60 using at least one of a general gas (for example, air) and vibration. For reference, in the present invention, a nutrient of a granule type not a powdery type or a liquid type may be supplied from the respective supply units 30 and, thus, the hopper 60 may be effectively cleaned simply by spraying a gas or generating vibration not by spraying a separate liquid (for example, water).

The packing unit 70 is provided to pack nutrient granules supplied from the respective supply units 30 into units of one dose. The packing unit 70 is provided to pack nutrient granules, agitated through various methods according to requirements and design specifications, into predetermined units, but the present invention is not limited as to the structure and method of the packing unit 70.

For example, with reference to FIG. 8, the packing unit 70 may include a film supply unit 72 to continuously supply a film, a guide unit 73 to guide the agitated nutrient granules into a plurality of packing spaces provided on the film, an adhesion unit 74 to close openings of the packing spaces, and a cutting unit 75 to cut the film into respective units corresponding to the packing spaces.

The film supply unit 72 may include a winding roller to wind the film in a roll type and a guide roller to guide the film supplied from the winding roller, but the present invention is not limited as to the structure and characteristics of the film supply unit 72. Although the embodiment of the present invention describes that the film supplied from the winding roller is guided by the guide roller under the condition that the direction of the film is changed by about 90 degrees by the guide roller, the film may be guided at different angles and through different methods according to requirements and design specifications.

Further, the film supply unit 72 may include a film sensor 710 to sense the amount of the film remaining on the winding roller and various sensors which may sense the remaining amount of the film may be used as the film sensor.

Further, a plurality of independently divided packing spaces may be provided on the film. The packing spaces may be formed while the film is supplied from the above-described film supply unit 72 or the film on which the packing spaces are formed already may be supplied from the film supply unit 72.

The guide unit 73 is provided to guide the agitated nutrient granules to the packing spaces provided on the film.

For example, the guide unit 73 may be provided adjacent to the outlet of the hopper 60 so as to rectilinearly move in the vertical direction and thus guide nutrient granules gathered in the hopper 60 to the openings of the packing spaces. Further, the guide unit 73 may include a knife 73a to widen the opening of the packing space.

The adhesion unit 74 is provided to close the opening of the packing space after the packing space is filled with nutrient granules. The adhesion unit 74 may be provided to seal the opening of the packing space using a general heat sealing method and, as circumstances require, be provided to seal the opening of the packing space using a general adhesive.

The cutting unit 75 may be provided to cut the film into respective units corresponding to the packing spaces and various general cutting devices may be used as the cutting unit 75 according to requirements and design specifications.

Such a packed nutrient may be shown in FIG. 9(a), and the packed nutrient may be discharged to the outside through the discharge conveyer 91 of the above-described table 90.

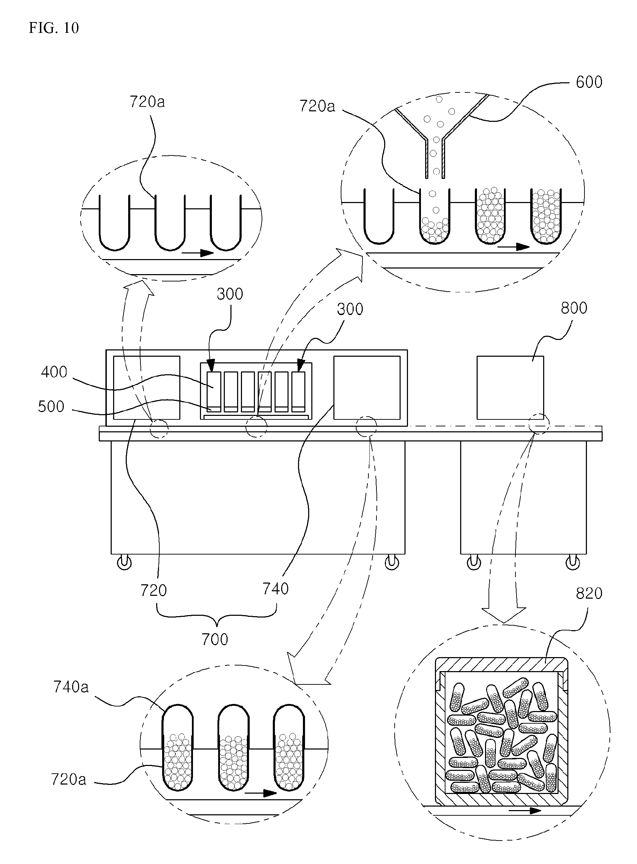

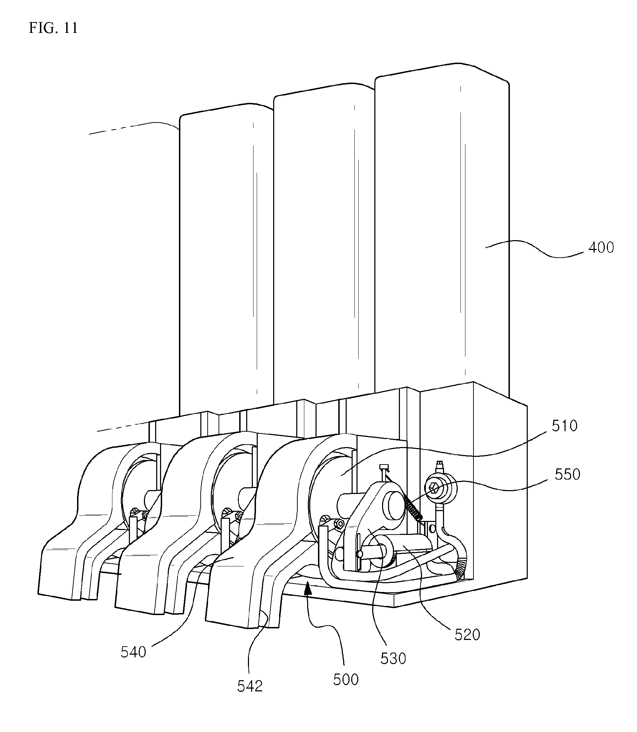

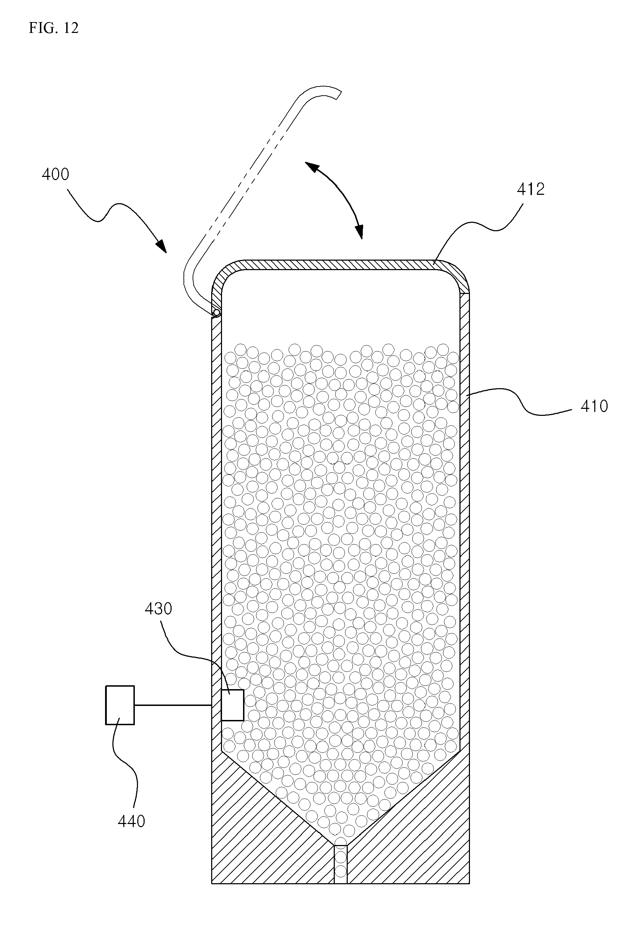

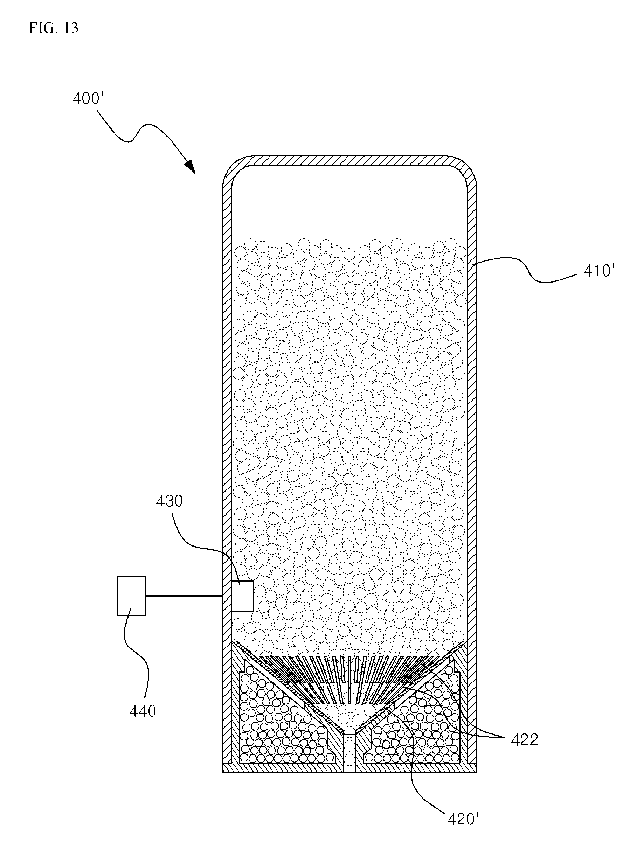

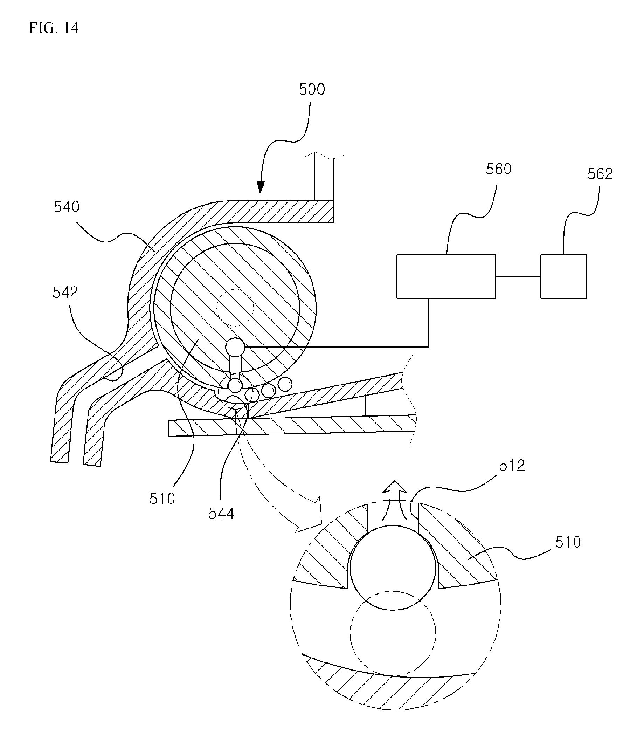

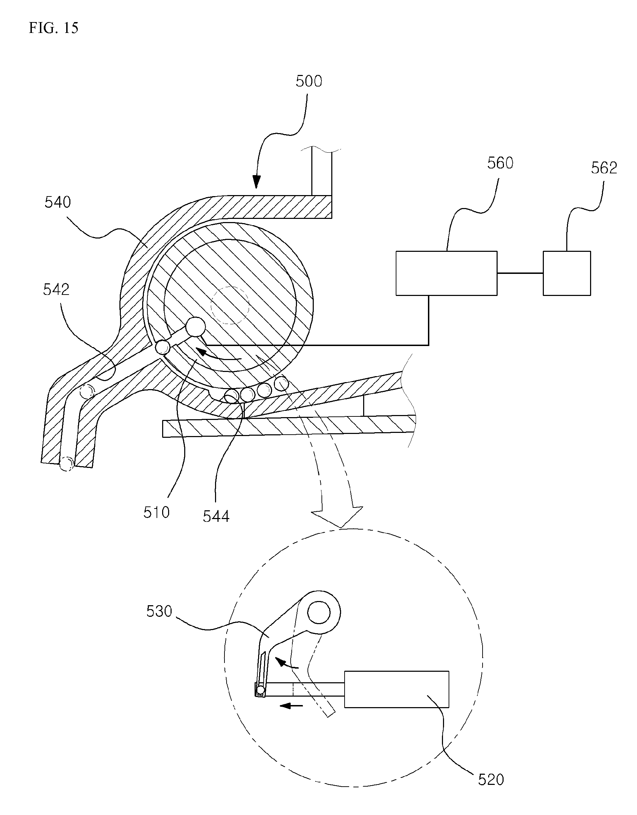

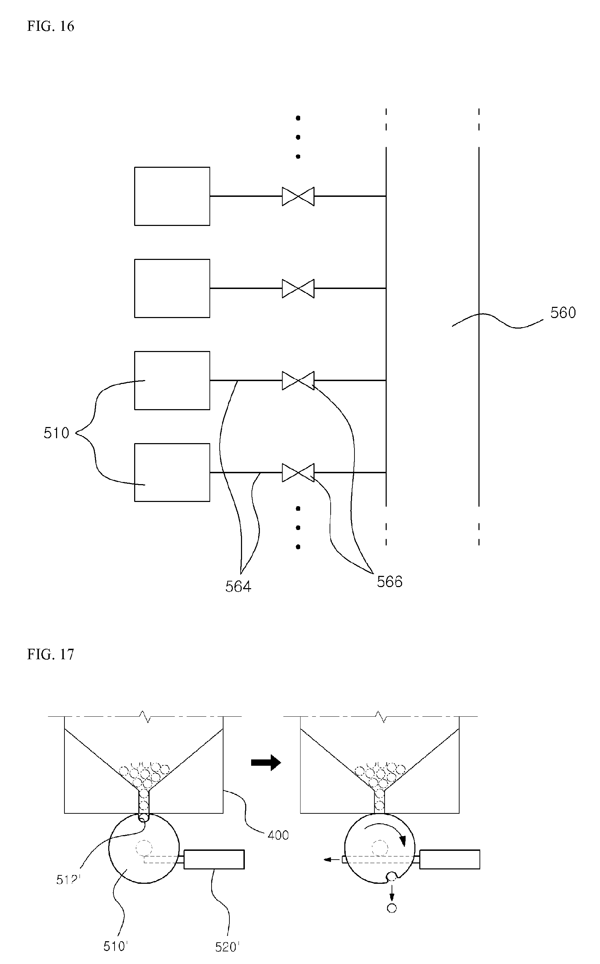

FIG. 10 is a view illustrating an individualized nutrient preparation apparatus in accordance with another embodiment of the present invention. FIG. 11 is a view illustrating a supply unit of the individualized nutrient preparation apparatus in accordance with another embodiment of the present invention, FIG. 12 is a view illustrating a storage part of the individualized nutrient preparation apparatus in accordance with another embodiment of the present invention, FIG. 13 is a view illustrating a modification of the storage part of the individualized nutrient preparation apparatus in accordance with another embodiment of the present invention, FIGs. 14 and 15 are views illustrating an operating structure of the supply unit of the individualized nutrient preparation apparatus in accordance with another embodiment of the present invention, FIG. 16 is a view illustrating a vacuum suction part of the individualized nutrient preparation apparatus in accordance with another embodiment of the present invention, and FIG. 17 is a view illustrating a modification of the supply unit of the individualized nutrient preparation apparatus in accordance with another embodiment of the present invention. Further, some parts in this embodiment which are substantially the same as those in the above-described former embodiment are denoted by the same reference numerals even though they are depicted in different drawings and a detailed description thereof will thus be omitted because it is considered to be unnecessary.

With reference to FIGs. 10 to 17, an individualized nutrient preparation apparatus in accordance with this embodiment of the present invention includes a data processing unit (not shown), supply units 300, each of which includes a storage part 400 and a discharge part 500, and a packing unit 700, and the discharge part 500 may include a pickup roller 510 and an actuator 520.

With reference to FIG. 12, the storage part 400 may be fixedly installed on an dedicated table (with reference to 90 of FIG. 1). For example, the storage part 400 may include a storage container 410 and an opening/closing member 420.

The storage container 410 may be formed in various shapes, such as a circular shape, a rectangular shape or a polygonal shape, according to requirements and design specifications. An opening may be formed at the upper portion of the storage container 410 and a discharge hole to discharge nutrient granules may be formed at the lower portion of the storage container 410.

The opening/closing member 420 may be provided to open and close the opening formed at the upper portion of the storage container 410. For example, the opening/closing member 420 may be provided at the upper portion of the storage container 410 so as to be rotatable about one end thereof and thus to be opened and closed. As circumstances require, the opening/closing member may be opened and closed using a general sliding method, but the present invention is not limited as to the opening and closing method of the opening/closing member.

If nutrient granules stored in the storage container 410 are less than a designated amount, the opening/closing member 420 may be opened so as to fill the storage container 410 with nutrient granules.

Further, a storage amount sensor 430 to sense the amount of nutrient granules stored in the storage container 410 may be provided in the storage container 410. A general sensor which may sense the amount of stored nutrient granules may be used as the storage amount sensor 430, but the present invention is not limited as to the kind and characteristics of the sensor.

A warning generation part 440 to output a warning signal if a value measured by the storage amount sensor 430 is less than a predetermined value may be provided. Therefore, if the amount of the stored nutrient granules is less than a designated reference, a manager may rapidly recognize this fact and replenish nutrient granules and the nutrient granules may be continuously supplied without interruption.

Here, the warning signal may include at least one of an audible warning signal by a sound unit and a visual warning signal by a general warning light, and various other waning signals recognizable by a user may be used.

With reference to FIG. 13, the storage part 400' may be mounted on the dedicated table so as to be selectively separated from the dedicated table and be replaced with different storage parts 400' according to requirements. For example, the storage part may include a storage container 410' and a partition member 420'.

The storage container 410' may be formed in various shapes, such as a circular shape, a rectangular shape or a polygonal shape, according to requirements and design specifications. A discharge hole (not shown) to discharge nutrient granules may be formed at the lower portion of the storage container 410' and an opening formed at the lower portion of the storage container 410' may be closed by a lower cap (not shown).

The partition member 420' may be provided within the storage container 410' and divide the inner space of the storage container 410' into a storage space to store nutrient granules and a reception space to receive a dehumidifying agent. Further, a plurality of communication slots 422' may be formed on the partition member 420' and the storage space and the reception space may communicate with each other by the communication slots 422'.

The partition member 420' may be provided in various shapes according to requirements and design specifications. For example, the partition member 420' may be formed in a kind of cone or funnel shape and the nutrient granules may be discharged to the outside of the storage container 410' through the discharge hole along the inclined surface of the partition member 420'.

The dehumidifying agent may be received in the reception space divided by the partition member 420', and a general dehumidifying agent, such as silica gel, may be used. As circumstances require, different dehumidifying agents may be used, but the present invention is not limited as to the kind and characteristics of the dehumidifying agent.

With reference to FIG. 11, the discharge part 50 includes the pickup roller 510 and the actuator 520 and, as the pickup roller 510 is rotated, nutrient granule may be picked up one by one by a pickup hole 512 and discharged to the outside. Further, the discharge part 50 may include a vacuum suction part 560 and, hereinafter, pickup of a nutrient granule by the pickup hole 512 using a vacuum suction method using vacuum suction force of the vacuum suction part 560 will be exemplarily described.

With reference to FIGs. 14 and 15, the pickup roller 510 may be provided to be rotatable adjacent to an outlet of the storage part 400, and the pickup hole 512 to pick up a nutrient granule may be formed on the outer circumferential surface of the pickup roller 510.

The pickup hole 512 may be formed to have a size to pick up one nutrient granule at a time and the number and separation interval of pickup holes 512 may be variously modified. Hereinafter, the pickup hole 512 which is disposed at the lower portion of the pickup roller 510 in the initial position of the pickup roller 510 (in a state prior to pickup) and has a smaller size (diameter) than nutrient granules so as to suck one nutrient granule at a time will be exemplarily described. As circumstances require, the pickup hole may have a size corresponding to nutrient granules.

The actuator 520 may provide driving force to rotate the pickup roller 510. Various types of actuators 520 may be used as the actuator 520 according to requirements and design specifications, but the present invention is not limited as to the connection structure between the actuator 520 and the pickup roller 510.

Hereinafter, an example in which the actuator 520 is configured to provide rectilinear driving force and an interlock member 530 to convert the rectilinear driving force into rotary driving force is connected to the pickup roller 510 will be described. For example, since a general solenoid providing rectilinear driving force may be used as the actuator 520 and the interlock member 530 may be connected to the side surface of the pickup roller 510 so as to be rotatable integrally with the pickup roller 510, the interlock member 530 is rotated and pushed by the rectilinear driving force of the actuator 520 and the pickup roller 510 may be rotated together with the interlock member 530. For this purpose, a long hole may be formed in the vertical direction on the interlock member 530 and a movable part of the actuator 520 may move along the long hole of the interlock member.

Further, a return spring 550 may be connected to the interlock member 530 and, after pickup of a nutrient granule by the pickup roller 510 has been completed, the interlock member 530 may be returned to an initial position thereof (the state prior to pickup) by the elastic force of the return spring 550 and the pickup roller 510 may also be returned to an initial position thereof. As circumstances require, the interlock member and the pickup roller may be configured to be returned to initial positions thereof by the restoring force of the actuator without the return spring.

Further, the discharge part 50 may include a transfer guide part 540 to transfer the nutrient granule picked up by the pickup roller 510. For example, the transfer guide part 540 may be formed to surround the outlet of the storage part 400 and the pickup roller 510 and a granule transfer hole 542 to transfer nutrient granules one by one may be formed on the transfer guide part 540. The granule transfer hole 542 may be inclined downwards toward the outlet and the nutrient granules introduced into the granule transfer hole 542 may be transferred by the inclination of the granule transfer hole 542. As circumstances require, the nutrient granule picked up by the pickup roller 510 may be directly transferred without the separate transfer guide.

Further, a temporary loader part 544 to temporarily load nutrient granules supplied from the storage part 400 thereon prior to pickup may be formed on the transfer guide part 540, and the nutrient granules supplied from the storage part 400 and temporarily loaded on the temporary loader part 544 may be picked up one by one by the pickup roller 510 and transferred through the granule transfer hole 542.

The vacuum suction part 560 may be prepared to provide vacuum suction force so that the pickup hole 512 may suck the nutrient granule through vacuum and a general vacuum pump may be used as the vacuum suction part 560.

Further, a vacuum pressure sensor 562 to sense vacuum pressure of the vacuum suction part 560 may be provided. A general sensor which may sense vacuum pressure of the vacuum suction part 560 may be used as the vacuum pressure sensor 562 and whether or not the nutrient granule is normally picked up may be sensed using a signal sensed by the vacuum pressure sensor 562. For example, when the vacuum pressure sensor 562 senses a predetermined pressure, it may be judged that a nutrient granule is normally picked up through the pickup hole 512 and, when the vacuum pressure sensor 562 senses a pressure lower than the predetermined pressure, it may be judged that a nutrient granule is abnormally picked up through the pickup hole 512. Further, if a nutrient granule is abnormally picked up through the pickup hole 512, pickup of the nutrient granule may be additionally performed manually or by automatic manipulation.

The vacuum suction part 560 may be individually provided on each of the supply units 30 or the supply units 30 may commonly use one vacuum suction part 560. With reference to FIG. 16, the vacuum suction part 560 and the pickup rollers 510 of the respective supply units 30 may be independently connected by a plurality of connection channels 564, and control valves 566 to selectively cut off independently at least one of the respective connection channels 564 may be provided on the respective connection channels 564. By such a structure, while a specific pickup roller 510 performs pickup of a nutrient granule through vacuum suction, other pickup rollers 510 may cut off the connection channels 564 through the control valves 566 and stop pickup of nutrient granules and, in such a manner, the frequencies of pickup of nutrient granules by the pickup rollers 510 of the respective supply units 30 may be independently adjusted properly.

Although the above-described embodiment of the present invention exemplarily describes the pickup rollers as picking up nutrient granules using a vacuum suction method, as circumstances require, nutrient granules may be picked up one by one simply by the pickup rollers without the vacuum suction part.

With reference to FIG. 17, a pickup roller 510' may be provided to be rotatable adjacent to the outlet of the storage part 400, and a pickup hole 512' to receive one nutrient granule at a time may be formed on the outer circumferential surface of the pickup roller 510'.

The pickup hole 512' may be disposed at the upper portion of the pickup roller 510' in the initial position of the pickup roller 510' (in a state prior to pickup) and a nutrient granule dropped from the outlet of the storage part 400 may be received in the pickup hole 512'. Thereafter, as the pickup roller 510 is rotated by a designated degree or more, the nutrient granule received in the pickup hole 512' may be discharged to the outside of the pickup hole 512'. Although FIG. 17 exemplarily describes one pickup hole 512' formed on the pickup roller 510', for reference, as circumstances require, a plurality of pickup holes may be formed on the pickup roller so as to be separated from one another and, in this case, sequentially pick up nutrient granules when the pickup roller is rotated.

Further, the pickup roller 510' may be configured so as to be rotated by rectilinear driving force of an actuator 520' through a rack and pinion method. As circumstances require, the pickup roller may be rotated by rectilinear driving force of the actuator using a separate interlock member, in the same manner as the above-described embodiment.

Although the above-described embodiment of the present invention exemplarily describes that nutrient granules are supplied one by one using the pickup roller, as circumstances require, nutrient granules may be supplied one by one using a slide opening/closing member or a rotary opening/closing member.

Referring to FIG. 10 again, the packing unit 700 may be configured to pack nutrient granules supplied from the respective supply units 300 into capsule units of one dose. The packing unit 700 may include a lower capsule supply unit 720 to supply lower capsules 720a and an upper capsule supply unit 740 to supply upper capsules 740a, and the upper capsule 740a may be combined with the upper portion of the lower capsulate 720a under the condition that the lower capsule 720a is filled with nutrient granules gathered by the hopper 600. Such a packed nutrient may be shown in FIG. 9(b).

Further, the packed nutrient capsules may be packed into a separated pack ing container 820 of a specific dose by a container packing unit 800. For example, the container packing unit 800 may pack the packed nutrient capsules into the packing container 820 of a weekly dose. A general packing container, such as a bottle or a barrel, may be used as the packing container 820, but the present invention is not limited as to the kind and characteristics of the packing container.

Further, although the above-described embodiment of the present invention exemplarily describes the packing unit as packing agitated nutrient granules into packets or capsules, the agitated nutrient granules may be packed into other types, such as sticks (c), as exemplarily shown in FIG. 9.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An individualized nutrient preparation apparatus to prepare a nutrient according to user characteristics, comprising:
a data processing unit configured to generate individual nutrient prescription data;
a plurality of supply units configured to independently supply different nutrient granules according to the individual nutrient prescription data, each of which supplying a predetermined number set of the nutrient granules; and
a packing unit configured to pack a mix of the nutrient granules.

2. The individualized nutrient preparation apparatus according to claim 1, wherein:
each of the supply units supplies the nutrient granules one by one as the predetermined number set to one dose; and
the packing unit packs the nutrient granules into units of one dose.

3. The individualized nutrient preparation apparatus according to claim 1, wherein each of the supply units includes:
a storage part to store the nutrient granules; and
a discharge part to discharge the nutrient granules one by one from the storage part.

4. The individualized nutrient preparation apparatus according to claim 3, wherein the discharge part includes:
a pickup plate including receipt grooves formed along the outer circumferential surface thereof and configured to be rotatable vertically so as to be adjacent to an outlet of the storage part; and
a guide member including a granule discharge hole and configured to surround the outer circumferential surface of the pickup plate,
wherein the nutrient granules are picked up one by one by the receipt grooves as the pickup plate is vertically rotated and, if the granule discharge hole is disposed so as to coincide with the receipt groove in the vertical direction when the pickup plate is rotated, the nutrient granule received in the receipt groove is discharged through the granule discharge hole.

5. The individualized nutrient preparation apparatus according to claim 3, wherein the discharge part includes:
a first plate including receipt grooves to receive the nutrient granules supplied from the storage part one by one; and
a second plate including a granule discharge hole and provided under the first plate so as to be rotatable relative to the first plate,
wherein, if the granule discharge hole is disposed so as to coincide with the receipt groove in the vertical direction during relative rotation between the first plate and the second plate, the nutrient granule received in the receipt groove is discharged through the granule discharge hole.

6. The individualized nutrient preparation apparatus according to claim 5, further comprising a discharge member provided adjacent to the granule discharge hole and configured to discharge the nutrient granule from the receipt groove approaching the granule discharge hole.

7. The individualized nutrient preparation apparatus according to claim 6, wherein:
guide grooves communicating with the receipt grooves are formed on the first plate;
a part of the discharge member is received in the guide grooves; and
the nutrient granule received in the receipt groove approaching the granule discharge hole is pressed by the discharge member and discharged.

8. The individualized nutrient preparation apparatus according to claim 4 or 5, further comprising:
a first sensor provided adjacent to one end of the granule discharge hole; and
a second sensor provided adjacent to the other end of the granule discharge hole, wherein:
the first sensor senses whether or not the receipt groove approaching the granule discharge hole receives the nutrient granule; and
the second sensor senses whether or not the receipt groove passing through the granule discharge hole discharges the nutrient granule.

9. The individualized nutrient preparation apparatus according to claim 3, wherein the discharge part includes:
a pickup roller including pickup holes and configured to be rotatable adjacent to the outlet of the storage part; and
an actuator configured to provide driving force to rotate the pickup roller, wherein, as the pickup roller is rotated, the nutrient granules are picked up one by one by the pickup holes and then discharged.

10. The individualized nutrient preparation apparatus according to claim 9, further comprising an interlock member connected to the pickup roller,
wherein the actuator provides rectilinear driving force and the interlock member converts the rectilinear driving force of the actuator into rotary driving force.

11. The individualized nutrient preparation apparatus according to claim 9, further comprising a transfer guide part configured to transfer the nutrient granules picked up by the pickup roller,
wherein a granule transfer hole to transfer the nutrient granules one by one is formed on the transfer guide part.

12. The individualized nutrient preparation apparatus according to claim 11, wherein a temporary loader part to temporarily load the nutrient granules prior to pickup is formed on the transfer guide part,
wherein the nutrient granules loaded on the temporary loader part are picked up by the pickup roller and transferred through the granule transfer hole.

13. The individualized nutrient preparation apparatus according to claim 9, wherein the discharge part further includes a vacuum suction part to provide vacuum suction force so that the nutrient granules are sucked by the pickup hole through vacuum.

14. The individualized nutrient preparation apparatus according to claim 13, further comprising a vacuum pressure sensor to sense the vacuum pressure of the vacuum suction part,
wherein whether or not the nutrient granule is picked up is sensed using a signal sensed by the vacuum pressure sensor.

15. The individualized nutrient preparation apparatus according to claim 13, further comprising:
a plurality connection channels configured to independently connect the vacuum suction part and the pickup rollers of the respective supply units; and
control valves provided on the respective connection channels to selectively cut off at least one of the connection channels independently.

16. The individualized nutrient preparation apparatus according to claim 3, wherein the storage part includes:
a storage container; and
an opening/closing member to open and close an opening formed at the upper portion of the storage container.

17. The individualized nutrient preparation apparatus according to claim 16, wherein:
guide plates to define the flow path of the nutrient granules are provided within the storage container; and
the nutrient granules are guided to the discharge part along the guide plates.

18. The individualized nutrient preparation apparatus according to claim 3, wherein the storage part includes:
a storage container; and
a partition member configured to divide the inner space of the storage container into a storage space to store the nutrient granules and a reception space to receive a dehumidifying agent and provided with communication slots to communicate the storage space and the reception space with each other.

19. The individualized nutrient preparation apparatus according to claim 3, further comprising a storage amount sensor configured to sense the amount of the nutrient granules stored in the storage part.

20. The individualized nutrient preparation apparatus according to claim 19, further comprising a warning generation part configured to output a warning signal if a value measured by the storage amount sensor is less than a predetermined value.

21. The individualized nutrient preparation apparatus according to claim 1, further comprising a hopper configured to gather the nutrient granules supplied from the respective supply units into one place.

22. The individualized nutrient preparation apparatus according to claim 1, wherein the packing unit packs the nutrient granules into at least one type of a capsule, a packet and a stick.

23. The individualized nutrient preparation apparatus according to claim 3, further comprising an air blowing device to dehumidify the nutrient granules stored in the storage part and a means of filtering to prevent influx of moisture in the air into the storage part.

24. The individualized nutrient preparation apparatus according to claim 1, wherein the data processing unit generates the individual nutrient prescription data corresponding to medical inquiry data according to individual characteristics based on predetermined reference data.

25. The individualized nutrient preparation apparatus according to claim 24, wherein the data processing unit compares the medical inquiry data with the reference data and calculates information regarding kinds and contents of specific nutrient ingredients required by an individual according to a result of comparison.

26. The individualized nutrient preparation apparatus according to claim 24, wherein the reference data is provided based on one recommended dose and the contents of the specific nutrient ingredients are increased or decreased according to the medical inquiry data according to individual characteristics.

27. The individualized nutrient preparation apparatus according to claim 26, wherein the medical inquiry data includes data regarding at least one of user's age, sex, weight, height, eating habits, drinking, smoking, labor type, possessing diseases and sleeping habits.

28. The individualized nutrient preparation apparatus according to claim 1, wherein the individual nutrient prescription data includes the composition ratio of at least one of vitamins, minerals, amino acids and a Korean traditional medicine.

29. An individualized nutrient preparation system to prepare a nutrient according user characteristics, comprising:
an input unit configured to input medical inquiry data according to individual characteristics;
a data processing unit configured to generate individual nutrient prescription data corresponding to the medical inquiry data; and
a preparation unit configured to receive the individual nutrient prescription data and prepare the nutrient according to the individual nutrient prescription data.

30. The individualized nutrient preparation system according to claim 29, wherein the data processing unit generates the individual nutrient prescription data corresponding to the medical inquiry data based on predetermined reference data.

31. The individualized nutrient preparation system according to claim 30, wherein the data processing unit compares the medical inquiry data with the reference data and calculates information regarding kinds and contents of specific nutrient ingredients required by an individual according to a result of comparison.

32. The individualized nutrient preparation system according to claim 31, wherein the reference data is provided based on a recommended daily dose or a single recommended dose and the contents of the specific nutrient ingredients are increased or decreased according to the medical inquiry data according to individual characteristics.

33. The individualized nutrient preparation system according to claim 29, wherein the input unit is configured to execute remote communication with the data processing unit.

34. The individualized nutrient preparation system according to claim 29, wherein:
the medical inquiry data includes data regarding at least one of user's age, sex, weight, height, eating habits, drinking, smoking, labor type, possessing diseases and sleeping habits; and
the individual nutrient prescription data includes the composition ratio of at least one of vitamins, minerals and amino acids.
